# EUROPÄISCHE PATENTSCHRIFT

(11) **EP 1 108 708 B1**
(45) Veröffentlichungstag und Bekanntmachung des Hinweises auf die Patenterteilung: **08.01.2003**
(21) Anmeldenummer: 00121908.8
(22) Anmeldetag: 07.10.2000
(51) Int. Cl.: C07C 211/53, A61K 7/13, C07C 217/84

(54) **Neue 1,4-Diaminobenzol-Derivate und diese Verbindungen enthaltende Färbemittel**
1,4-Diaminobenzene derivatives and colouring agents containing these compounds
Dérivés du 1,4-diaminobenzène et colorants contenant ces composés

(30) Priorität: 18.12.1999 DE 19961274
(43) Veröffentlichungstag der Anmeldung: 20.06.2001
(73) Patentinhaber: Wella Aktiengesellschaft, 64274 Darmstadt (DE)
(72) Erfinder: Chassot, Laurent, 1724 Praroman (CH); Braun, Hans-Jürgen, 3182 Überstorf (CH)

(56) Entgegenhaltungen:
- EP-A- 0 173 932
- EP-A- 0 195 361
- WO-A-99/59527

## Beschreibung

Die Erfindung betrifft neue 2,5-Diamino-1-aminomethylbenzol -Derivate sowie diese Verbindungen enthaltende Mittel zum Färben von Keratinfasern, insbesondere menschlichen Haaren.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen allein oder mit bestimmten Kupplersubstanzen in Gegenwart eines geeigneten Oxidationsmittels. So ist es aus der DE-PS 47349 bekannt, dass Keratinfasern durch p-Phenylendiamin in Abhängigkeit vom verwendeten Oxidationsmittel in hellblonden bis blauschwarzen Farbtönen gefärbt werden können. Zur Erzeugung von blauen Farbtönen werden jedoch Kombinationen von p-Phenylendiamin beziehungsweise desssen Derivaten mit bestimmten Kupplersubstanzen benötigt. Da für die Haarfärbung nur Verbindungen eingesetzt werden können, die einerseits den Anforderung zum Schutz der Verbraucher genügen und anderseits über einen längeren Zeitraum beständige Färbungen ergeben, ist die Auswahl an geeigneten Entwicklersubstanzen und Kupplersubstanzen beschränkt.

Es wurde nun überraschend gefunden, dass bestimmte p-Phenylendiaminderivate auch ohne Zusatz von sogenannten Kupplersubstanzen eine intensiv blaue Färbung von Fasern, insbesondere Keratinfasem wie zum Beispiel Haaren, ermöglichen.

So werden bei Verwendung dieser p-Phenylendiaminderivate in oxidierendem Medium farbstarke Farbnuancen erhalten, die außerordentlich lichtecht und waschecht sind.

Gegenstand der vorliegende Erfindung sind daher 2,5-Diamino-1-aminomethylbenzol-Derivate der allgemeinen Formel (I) oder deren physiologisch verträgliche, wasserlösliche Salze, 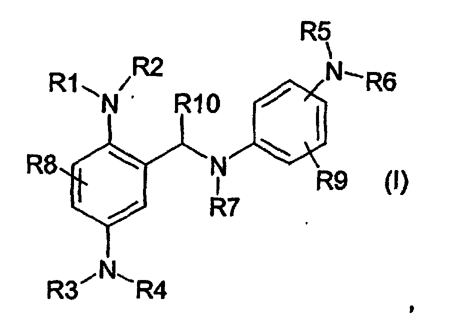 worin
**R1,R2,R3,R4,R5,R6 und R7** unabhängig voneinander Wasserstoff, eine C₁-C₆- Alkylgruppe, eine C₁-C₄-Hydroxyalkylgruppe, eine C₂-C₄-Dihydroxyalkylgruppe oder eine C₁-C₄-Alkoxy-(C₁-C₂)-alkylgruppe darstellen oder R1 und R2 und/oder R3 und R4 und/oder R5 und R6 gemeinsam einen viergliedrigen bis achtgliedrigen aliphatischen Ring bilden, unter der Bedingung, dass mindestens 2 der Reste R1 bis R6 Wasserstoff darstellen;
**R8** gleich Wasserstoff, einem Halogenatom, einer C₁-C₄-Alkylgruppe, einer C₁-C₄-Hydroxyalkylgruppe oder einer C₁-C₄-Alkoxygruppe ist;
**R9** Wasserstoff, ein Halogenatom, eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄-Alkylthioethergruppe, eine Mercaptogruppe, eine Aminogruppe, eine C₁-C₄-Alkylaminogruppe, eine C₁-C₄-Hydroxyalkyl-aminogruppe, eine Di(C₁-C₄)-alkylaminogruppe, eine Di(C₁-C₄-hydroxyalkyl)-aminogruppe, eine (Dihydroxy(C₂-C₄)-alkyl)-aminogruppe, eine (C₁-C₄-Hydroxyalkyl)-C₁-C₄-alkylaminogruppe, eine Trifluormethangruppe, eine C₁-C₄-Hydroxyalkylgruppe oder eine C₃-C₄ Dihydroxyalkylgruppe bedeutet; und
**R10** gleich Wasserstoff oder einer C₁-C₆ Alkylgruppe Gruppe ist.

Als Verbindungen der Formel (1) können beispielweise die folgenden Verbindungen genannt werden:
2-((2-Amino-phenylamino)-methyl)-1,4-diamino-benzol; 2-((2-Bis(hydroxyethyl)amino-phenylamino)-methyl)-1,4-diamino-benzol; 2-((2-Dimethylamino-phenylamino)-methyl)-1,4-diamino-benzol; 2-((3-aminophenylamino)-methyl)-1,4-diamino-benzol; 2-((3-Bis(hydroxyethyl)aminophenylamino)-methyl)-1,4-diamino-benzol; 2-((3-Dimethylaminophenylamino)-methyl) -1,4-diamino-benzol; 2-((4-Amino-phenylamino)-methyl) -1,4-diamino-benzol; 2-((4-Bis(hydroxyethyl)amino-phenylamino)-methyl) -1,4-diamino-benzol; 2-((4-Dimethylamino-phenylamino)-methyl)-1,4-diamino-benzol; N¹,N¹-Bis(hydroxyethyl)-2-((2-amino-phenylamino)-methyl) -1,4-diamino-benzol; N¹, N¹-Bis(hydroxyethyl)-2-((2-bis(hydroxyethyl)amino-phenylamino)-methyl)-1,4-diamino-benzol; N¹,N¹-Bis-(hydroxyethyl)-2-((2-dimethylamino-phenylamino)-methyl)-1,4-diaminobenzol; N¹,N¹-Bis(hydroxyethyl)-2-((3-amino-phenylamino)-methyl)-1,4-diamino-benzol; N¹, N¹-Bis(hydroxyethyl)-2-((3-bis(hydroxyethyl)aminophenylamino)-methyl) -1,4-diamino-benzol; N¹,N¹-Bis(hydroxyethyl)-2-((3-dimethylamino-phenylamino)-methyl)-1,4-diamino-benzol; N¹, N¹-Bis-(hydroxyethyl)-2-((4-amino-phenylamino)-methyl)-1,4-diamino-benzol; N¹,N¹-Bis(hydroxyethyl)-2-((4-bis(hydroxyethyl)amino-phenylamino)-methyl)-1,4-diamino-benzol; N¹,N¹-Bis(hydroxyethyl)-2-((4-dimethylammo-phenylamino)-methyl) -1,4-diamino-benzol; N¹-Dihydroxypropyl-2-((2-amino-phenylamino)-methyl) -1,4-diamino-benzol; N¹-Dihydroxypropyl-2-((2-bis(hydroxyethyl)amino-phenylamino)-methyl)-1,4-diamino-benzol; N¹-Dihydroxypropyl-2-((2-dimethylamino-phenylamino)-methyl)-1,4-diamino-benzol; N¹-Dihydroxypropyl-2-((3-amino-phenylamino)-methyl)-1,4-diamino-benzol; N¹-Dihydroxypropyl-2-((3-bis(hydroxyethyl)aminophenylamino)-methyl) -1,4-diamino-benzol; N¹-Dihydroxypropyl-2-((3-dimethylamino-phenylamino)-methyl) -1,4-diamino-benzol; N¹-Dihydroxypropyl-2-((4-amino-phenylamino)-methyl)-1,4-diamino-benzol; N¹-Dihydroxypropyl-2-((4-bis(hydroxyethyl)amino-phenylamino)-methyl)-1,4-diamino-benzol; N¹-Dihydroxypropyl-2-((4-dimethylamino-phenylamino)-methyl)-1,4-diamino-benzol; N¹-Hydroxyethyl-2-((2-aminophenylamino)-methyl)-1,4-diamino-benzol; N¹-Hydroxyethyl-2-((2-bis(hydroxyethyl)amino-phenylamino)-methyl)-1,4-diamino-benzol; N¹-Hydroxyethyl-2-((2-dimethylamino-phenylamino)-methyl)-1,4-diaminobenzol; N¹-Hydroxyethyl-2-((3-amino-phenylamino)-methyl)-1,4-diaminobenzol; N¹-Hydroxyethyl-2-((3-bis(hydroxyethyl)amino-phenylamino)-methyl) -1,4-diamino-benzol; N¹-Hydroxyethyl-2-((3-dimethylaminophenylamino)-methyl) -1,4-diamino-benzol; N¹-Hydroxyethyl-2-((4-aminophenylamino)-methyl)-1,4-diamino-benzol; N¹-Hydroxyethyl-2-((4-bis(hydroxyethyl)amino-phenylamino)-methyl)-1,4-diamino-benzol; N¹-Hydroxyethyl-2-((4-dimethylamino-phenylamino)-methyl)-1,4-diaminobenzol; N⁴, N⁴-Bis(hydroxyethyl)-2-((2-amino-phenylamino)-methyl)-1,4-diamino-benzol; N⁴,N⁴-Bis(hydroxyethyl)-2-((2-bis(hydroxyethyl)aminophenylamino)-methyl)-1,4-diamino-benzol; N⁴, N⁴-Bis(hydroxyethyl)-2-((2-dimethylamino-phenylamino)-methyl)-1,4-diamino-benzol; N⁴, N⁴-Bis-(hydroxyethyl)-2-((3-amino-phenylamino)-methyl)-1,4-diamino-benzol; N⁴,N⁴-Bis(hydroxyethyl)-2-((3-bis(hydroxyethyl)amino-phenylamino)-methyl)-1,4-diamino-benzol; N⁴,N⁴-Bis(hydroxyethyl)-2-((3-dimethylamino-phenylamino)-methyl)-1,4-diamino-benzol; N⁴,N⁴-Bis(hydroxyethyl)-2-((4-amino-phenylamino)-methyl)-1,4-diamino-benzol; N⁴, N⁴-Bis(hydroxyethyl)-2-((4-bis(hydroxyethyl)amino-phenylamino)-methyl)-1,4-diamino-benzol; N⁴, N⁴-Bis(hydroxyethyl)-2-((4-dimethylamino-phenylamino)-methyl)-1,4-diamino-benzol; N⁴-Dihydroxypropyl-2-((2-amino-phenylamino)-methyl)-1,4-diamino-benzol; N⁴-Dihydroxypropyl-2-((2-bis(hydroxyethyl)amino-phenylamino)-methyl) -1,4-diamino-benzol; N⁴-Dihydroxypropyl-2-((2-dimethylamino-phenylamino)-methyl) -1,4-diamino-benzol; N⁴-Dihydroxypropyl-2-((3-amino-phenylamino)-methyl)-1,4-diamino-benzol; N⁴-Dihydroxypropyl-2-((3-bis(hydroxyethyl)aminophenylamino)-methyl) -1,4-diamino-benzol; N⁴-Dihydroxypropyl-2-((3-dimethylamino-phenylamino)-methyl) -1,4-diamino-benzol; N⁴-Dihydroxypropyl-2-((4-amino-phenylamino)-methyl) -1,4-diamino-benzol; N⁴-Dihydroxypropyl-2-((4-bis(hydroxyethyl)amino-phenylamino)-methyl)-1,4-diamino-benzol; N⁴-Dihydroxypropyl-2-((4-dimethylaminophenylamino)-methyl)-1,4-diamino-benzol; N⁴-Hydroxyethyl-2-((2-aminophenylamino)-methyl)-1,4-diamino-benzol; N⁴-Hydroxyethyl-2-((2-bis(hydroxyethyl)amino-phenylamino)-methyl)-1,4-diamino-benzol; N⁴-Hydroxyethyl-2-((2-dimethylamino-phenylamino)-methyl)-1,4-diaminobenzol; N⁴-Hydroxyethyl-2-((3-amino-phenylamino)-methyl)-1,4-diaminobenzol; N⁴-Hydroxyethyl-2-((3-bis(hydroxyethyl)amino-phenylamino)-methyl)-1,4-diamino-benzol; N⁴-Hydroxyethyl-2-((3-dimethylaminophenylamino)-methyl)-1,4-diamino-benzol; N⁴-Hydroxyethyl-2-((4-aminophenylamino)-methyl) -1,4-diamino-benzol; N⁴-Hydroxyethyl-2-((4-bis-(hydroxyethyl)amino-phenylamino)-methyl) -1,4-diamino-benzol; N⁴-Hydroxyethyl-2-((4-dimethylamino-phenylamino)-methyl)-1,4-diaminobenzol; 2-((2-(Dihydroxypropyl)amino-phenylamino)-methyl)-1,4-diaminobenzol; 2-((2-(Pyrrolidin-1-yl)-phenylamino)-methyl)-1,4-diamino-benzol; 2-((2-Hydroxyethylamino-phenylamino)-methyl)-1,4-diamino-benzol; 2-((2-Methylamino-phenylamino)-methyl)-1,4-diamino-benzol; 2-((3-(Dihydroxypropyl)amino-phenylamino)-methyl)-1,4-diamino-benzol; 2-((3-(Pyrrolidin-1-yl)-phenylamino)-methyl)-1,4-diamino-benzol; 2-((3-Hydroxyethylamino-phenylamino)-methyl)-1,4-diamino-benzol; 2-((3-Methylamino-phenylamino)-methyl) -1,4-diamino-benzol; 2-((4-(Dihydroxypropyl)amino-phenylamino)-methyl)-1,4-diamino-benzol; 2-((4-(Pyrrolidin-1-yl)-phenylamino)-methyl)-1,4-diamino-benzol; 2-((4-Hydroxyethylamino-phenylamino)-methyl)-1,4-diamino-benzol; 2-((4-Methylamino-phenylamino)-methyl)-1,4-diamino-benzol; 2-((Methyl-(2-(dihydroxypropylamino)-phenyl)-amino)-methyl)-1,4-diamino-benzol; 2-((Methyl-(2-amino-phenyl)-amino)-methyl)-1,4-diamino-benzol; 2-((Methyl-(2-bis(hydroxyethyl)amino-phenyl)-amino)-methyl)-1,4-diaminobenzol; 2-((Methyl-(3-(dihydroxypropylamino)-phenyl)-amino)-methyl) -1,4-diamino-benzol; 2-((Methyl-(3-amino-phenyl)-amino)-methyl)-1,4-diaminobenzol; 2-((Methyl-(3-bis(hydroxyethyl)amino-phenyl)-amino)-methyl) -1,4-diamino-benzol; 2-((Methyl-(4-(dihydroxypropylamino)-phenyl)-amino)-methyl) -1,4-diamino-benzol; 2-((Methyl-(4-amino-phenyl)-amino)-methyl)-1,4-diamino-benzol; 2-((Methyl-(4-bis(hydroxyethyl)amino-phenyl)-amino)-methyl) -1,4-diamino-benzol; 2-((1-(2-Amino-phenyl)-amino)-ethyl) -1,4-diamino-benzol; 2-((1-(2-Bis(hydroxyethyl)amino-phenyl)-amino)-ethyl)-1,4-diamino-benzol; 2-((1-(2-Dimethylamino-phenyl)-amino)-ethyl) -1,4-diamino-benzol; 2-((1-(3-Amino-phenyl)-amino)-ethyl)-1,4-diamino-benzol; 2-((1-(3-Bis(hydroxyethyl)amino-phenyl)-amino)-ethyl)-1,4-diaminobenzol; 2-((1-(3-Dimethylamino-phenyl)-amino)-ethyl)-1,4-diamino-benzol; 2-((1-(4-Amino-phenyl)-amino)-ethyl)-1,4-diamino-benzol; 2-((1-(4-Bis-(hydroxyethyl)amino-phenyl)-amino)-ethyl)-1,4-diamino-benzol; 2-((1-(4-Dimethylamino-phenyl)-amino)-ethyl)-1,4-diamino-benzol; 2-((2-((2,3-Diamino-phenylamino)-methyl)-1,4-diamino-benzol; 2-((2-((2,4-Diamino-phenylamino)-methyl)-1,4-diamlno-benzol; 2-((2-((3,4-Diamino-phenylamino)-methyl)-1,4-diamino-benzol; 2-((Methyl-(2,3-diamino-phenyl)-amino)-methyl)-1,4-diamino-benzol; 2-((Methyl-(2,4-diamino-phenyl)-amino)-methyl)-1,4-diamino-benzol; 2-((Methyl-(3,4-diamino-phenyl)-amino)-methyl)-1,4-diamino-benzol; 2-(1-(2,3-Diamino-phenylamino)-ethyl)-1,4-diamino-benzol; 2-(1-(2,4-Diamino-phenylamino)-ethyl)-1,4-diamino-benzol; 2-(1-(3,4-Diamino-phenylamino)-ethyl)-1,4-diamino-benzol; 2-((2-(2-Hydroxyethoxy)-4-amino-phenylamino)-methyl)-1,4-diaminobenzol; 2-((2-(2-Hydroxyethyl)-4-amino-phenylamino)-methyl)-1,4-diamino-benzol; 2-((2-Chlor-4-amino-phenylamino)-methyl)-1,4-diaminobenzol; 2-((2-Hydroxy-4-amino-phenylamino)-methyl)-1,4-diamino-benzol; 2-((2-Methoxy-4-amino-phenylamino)-methyl)-1,4-diamino-benzol; 2-((2-Methyl-4-amino-phenylamino)-methyl)-1,4-diamino-benzol; 2-((3-(2-Hydroxyethoxy)-4-amino-phenylamino)-methyl)-1,4-diaminobenzol; 2-((3-(2-Hydroxyethyl)-4-amino-phenylamino)-methyl)-1,4-diamino-benzol; 2-((3-Chlor-4-amino-phenylamino)-methyl)-1,4-diaminobenzol; 2-((3-Hydroxy-4-amino-phenylamino)-methyl)-1,4-diamino-benzol; 2-((3-Methoxy-4-amino-phenylamino)-methyl)-1,4-diamino-benzol; 2-((3-Methyl-4-amino-phenylamino)-methyl)-1,4-diamino-benzol; 2-((2-(2-Hydroxyethoxy)-3-amino-phenylamino)-methyl)-1,4-diaminobenzol; 2-((2-(2-Hydroxyethyl)-3-amino-phenylamino)-methyl)-1,4-diamino-benzol; 2-((2-Chlor-3-amino-phenylamino)-methyl)-1,4-diaminobenzol; 2-((2-Hydroxy-3-amino-phenylamino)-methyl)-1,4-diamino-benzol; 2-((2-Methyl-3-amino-phenylamino)-methyl)-1,4-diamino-benzol; 2-((4-(2-Hydroxyethoxy)-3-amino-phenylamino)-methyl)-1,4-diaminobenzol; 2-((4-(2-Hydroxyethyl)-3-amino-phenylamino)-methyl)-1,4-diamino-benzol; 2-((4-Chlor-3-amino-phenylamino)-methyl)-1,4-diaminobenzol; 2-((4-Hydroxy-3-amino-phenylamino)-methyl)-1,4-diamino-benzol; 2-((4-Methyl-3-amino-phenylamino)-methyl)-1,4-diamino-benzol; 2-((5-(2-Hydroxyethoxy)-3-amino-phenylamino)-methyl)-1,4-diaminobenzol; 2-((5-(2-Hydroxyethyl)-3-amino-phenylamino)-methyl)-1,4-diamino-benzol; 2-((5-Chlor-3-amino-phenylamino)-methyl)-1,4-diaminobenzol; 2-((5-Hydroxy-3-amino-phenylamino)-methyl)-1,4-diamino-benzol; 2-((5-Methyl-3-amino-phenylamino)-methyl)-1,4-diamino-benzol; 2-((6-(2-Hydroxyethoxy)-3-amino-phenylamino)-methyl)-1,4-diaminobenzol; 2-((6-(2-Hydroxyethyl)-3-amino-phenylamino)-methyl)-1,4-diamino-benzol; 2-((6-Chlor-3-amino-phenylamino)-methyl)-1,4-diaminobenzol; 2-((6-Hydroxy-3-amino-phenylamino)-methyl)-1,4-diamino-benzol; 2-((6-Methyl-3-amino-phenylamino)-methyl)-1,4-diamino-benzol; 2-((3-(2-Hydroxyethoxy)-2-amino-phenylamino)-methyl)-1,4-diaminobenzol; 2-((3-(2-Hydroxyethyl)-2-amino-phenylamino)-methyl)-1,4-diamino-benzol; 2-((3-Chlor-2-amino-phenylamino)-methyl)-1,4-diaminobenzol; 2-((3-Hydroxy-2-amino-phenylamino)-methyl)-1,4-diamino-benzol; 2-((3-Methoxy-2-amino-phenylamino)-methyl)-1,4-diamino-benzol; 2-((3-Methyl-2-amino-phenylamino)-methyl)-1,4-diamino-benzol; 2-((4-(2-Hydroxyethoxy)-2-amino-phenylamino)-methyl)-1,4-diaminobenzol; 2-((4-(2-Hydroxyethyl)-2-amino-phenylamino)-methyl)-1,4-diamino-benzol; 2-((4-Chlor-2-amino-phenylamino)-methyl)-1,4-diaminobenzol; 2-((4-Hydroxy-2-amino-phenylamino)-methyl)-1,4-diamino-benzol; 2-((4-Methoxy-2-amino-phenylamino)-methyl)-1,4-diamino-benzol; 2-((4-Methyl-2-amino-phenylamino)-methyl)-1,4-diamino-benzol; 2-((5-(2-Hydroxyethoxy)-2-amino-phenylamino)-methyl)-1,4-diaminobenzol; 2-((5-(2-Hydroxyethyl)-2-amino-phenylamino)-methyl)-1,4-diamino-benzol; 2-((5-Chlor-2-amino-phenylamino)-methyl)-1,4-diaminobenzol; 2-((5-Hydroxy-2-amino-phenylamino)-methyl)-1,4-diamino-benzol; 2-((5-Methoxy-2-amino-phenylamino)-methyl)-1,4-diamino-benzol; 2-((5-Methyl-2-amino-phenylamino)-methyl)-1,4-diamino-benzol; 2-((6-(2-Hydroxyethoxy)-2-amino-phenylamino)-methyl)-1,4-diaminobenzol; 2-((6-(2Hydroxyethyl)-2-amino-phenylamino)-methyl)-1,4-diamino-benzol; 2-((6-Chlor-2-amino-phenylamino)-methyl)-1,4-diaminobenzol; 2-((6-Hydroxy-2-amino-phenylamino)-methyl)-1,4-diamino-benzol; 2-((6-Methoxy-2-amino-phenylamino)-methyl)-1,4-diamino-benzol; 2-((6-Methyl-2-amino-phenylamino)-methyl)-1,4-diamino-benzol.

Bevorzugt sind Verbindungen der Formel (I), bei denen (i) **R1, R2, R3** und **R4** gleichzeitig Wasserstoff bedeuten und/oder (ii) einer oder mehrere der Reste **R5** bis **R10** gleich Wasserstoff sind und/oder (iii) **R5** und **R6** unabhängig voneinander gleich Wasserstoff, einer Methylgruppe oder einer C₁-C₄-Hydroxyalkylgruppe sind oder (iv) **R7, R8, R9** und **R10** gleich Wasserstoff sind und **R5** und **R6** unabhänging voneinander gleich Wasserstoff, einer C₁-C₄-Alkylgruppe, einer C₁-C₄- Hydroxyalkylgruppe oder einer C₂-C₄-Dihydroxyalkylgruppe sind oder **R5** und **R6** gemeinsam einen viergliedrigen Ring bilden.

Insbesondere sind die folgenden Verbindungen zu nennen: 2-((2-Amino-phenylamino)-methyl)-1,4-diamino-benzol; 2-((3-Aminophenylamino)-methyl)-1,4-diamino-benzol; 2-((4-Amino-phenylamino)-methyl)-1,4-diamino-benzol; 2-((4-Bis(2-hydroxyethyl)amino-phenylamino)-methyl)-1,4-diamino-benzol; 2-((4-Dimethylamino-phenylamino)-methyl)-1,4-diamino-benzol; N¹,N¹-Bis(hydroxyethyl)-2-((3-aminophenylamino)-methyl)-1,4-diamino-benzol; N¹,N¹-Bis(hydroxyethyl)-2-((4-amino-phenylamino)-methyl)-1,4-diamino-benzol; N¹,N¹-Bis(hydroxyethyl)-2-((4-bis(2-hydroxyethyl)amino-phenylamino)-methyl)-1,4-diamino-benzol; N¹-Dihydroxypropyl-2-((4-amino-phenylamino)-methyl)-1,4-diaminobenzol; N¹-Dihydroxypropyl-2-((4-bis(2-hydroxyethyl)amino-phenylamino)-methyl)-1,4-diamino-benzol; N¹-Dihydroxypropyl-2-((4-dimethylaminophenylamino)-methyl)-1,4-diamino-benzol; N⁴,N⁴-Bis(hydroxyethyl)-2-((3-amino-phenylamino)-methyl)-1,4-diamino-benzol; N⁴,N⁴-Bis(hydroxyethyl)-2-((4-amino-phenylamino)-methyl)-1,4-diamino-benzol; N⁴,N⁴-Bis(hydroxyethyl)-2-((4-bis(2-hydroxyethyl)amino-phenylamino)-methyl)-1,4-diaminobenzol; N⁴,N⁴-Bis(hydroxyethyl)-2-((4-dimethylamino-phenylamino)-methyl)-1,4-diamino-benzol; N⁴-Dihydroxypropyl-2-((4-amino-phenylamino)-methyl)-1,4-diamino-benzol; N⁴-Dihydroxypropyl-2-((4-bis(2-hydroxyethyl)amino-phenylamino)-methyl)-1,4-diamino-benzol; N⁴-Dihydroxypropyl-2-((4-dimethylamino-phenylamino)-methyl)-1,4-diamino-benzol; 2-((2-(2-Hydroxyethyl)amino-phenylamino)-methyl)-1,4-diamino-benzol; 2-((2-(Pyrrolidin-1-yl)-phenylamino)-methyl)-1,4-diaminobenzol; 2-((2-Dihydroxypropylamino-phenylamino)-methyl-1,4-diaminobenzol; 2-((2-Methylamino-phenylamino)-methyl)-1,4-diamino-benzol; 2-((3-(2-Hydroxyethyl)amino-phenylamino)-methyl)-1,4-diamino-benzol; 2-((3-(Pyrrolidin-1-yl)-phenylamino)-methyl)-1,4-diamino-benzol; 2-((3-Dihydroxypropylamino-phenylamino)-methyl)-1,4-diamino-benzol; 2-((3-Methylamino-phenylamino)-methyl)-1,4-diamino-benzol; 2-((4-(2-Hydroxyethyl)amino-phenylamino)-methyl)-1,4-diamino-benzol; 2-((4-(Pyrrolidin-1-yl)-phenylamino)-methyl)-1,4-diamino-benzol; 2-((4-Dihydroxypropylamino-phenylamino)-methyl)-1,4-diamino-benzol; 2-((4-Methylamino-phenylamino)-methyl)-1,4-diamino-benzol; 2-((2-(2-Hydroxyethyl)-4-amino-phenylamino)-methyl)-1,4-diamino-benzol; 2-((2-(2-Hydroxyethyl)-4-bis(2-hydroxyethyl)amino-phenylamino)-methyl)-1,4-diamino-benzol; 2-((2-Chlor-4-amino-phenylamino)-methyl)-1,4-diamino-benzol; 2-((2-Chlor-4-bis(2-hydroxyethyl)amino-phenylamino)-methyl)-1,4-diamino-benzol; 2-((2-Methoxy-4-amino-phenylamino)-methyl)-1,4-diamino-benzol; 2-((2-Methoxy-4-bis(2-hydroxyethyl)aminophenylamino)-methyl)-1,4-diamino-benzol; 2-((2-Methyl-4-aminophenylamino)-methyl)-1,4-diamino-benzol; 2-((2-Methyl-4-bis(2-hydroxyethyl)amino-phenylamino)-methyl)-1,4-diamin--benzol; 2-((3-(2-Hydroxyethyl)-4-amino-phenylamino)-methyl)-1,4-diamino-benzol; 2-((3-(2-Hydroxyethyl)-4-bis(2-hydroxyethyl)amino-phenylamino)-methyl)-1,4-diamino-benzol; 2-((3-Chlor-4-amino-phenylamino)-methyl)-1,4-diamino-benzol; 2-((3-Chlor-4-bis(2-hydroxyethyl)amino-phenylamino)-methyl-(1,4-diamino-benzol; 2-((3-Methoxy-4-amino-phenylamino)-methyl)-1,4-diamino-benzol; 2-((3-Methoxy-4-bis(2-hydroxyethyl)aminophenylamino)-methyl)-1,4-diamino-benzol; 2-((3-Methyl-4-aminophenylamino)-methyl)-1,4-diamino-benzol; 2-((3-Methyl-4-bis(2-hydroxyethyl)amino-phenylamino)-methyl)-1,4-diamino-benzol; 2-((4-(2-Hydroxyethoxy)-3-amino-phenylamino)-methyl)-1,4-diamino-benzol; 2-((4-(2-Hydroxyethoxy)-3-bis(2-hydroxyethyl)amino-phenylamino)-methyl)-1,4-diamino-benzol; 2-((4-(2-Hydroxyethyl)-3-aminophenylamino)-methyl)-1,4-diamino-benzol und 2-((4-(2-Hydroxyethyl)-3-bis(2-hydroxyethyl)amino-phenylamino)-methyl)-1,4-diamino-benzol.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen 2,5-Diamino-1-aminomethylbenzol-Derivate der Formel (I) kann unter Verwendung von bekannten Syntheseverfahren erfolgen. Die Synthese der erfindungsgemässen Verbindungen kann beispielsweise wie folgt durchgeführt werden: Entweder a) durch eine reduktive Aminierung eines substituierten Benzols der Formel (II) 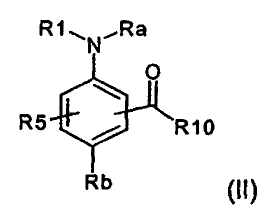 mit einem Amin der Formel (III) und anschliessende Abspaltung der Schutzgruppe; 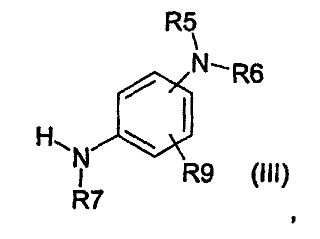 wobei in den Formeln (II) und (III) die Restgruppen die folgende Bedeutung haben:
**Ra** steht für eine Schutzgruppe, wie sie beispielsweise in dem Kapitel "Protective Groups in Organic Synthesis", Kapitel 7, Wiley Interscience, 1991 beschrieben wird;
**Rb** ist gleich NR1 Ra oder NR1R2, während
**X, R1, R2, R5, R6, R7, R9** und **R10** die in Formel (I) gennante Bedeutung haben; oder

### b) durch Substition eines substituierten Benzols der Formel (IV)

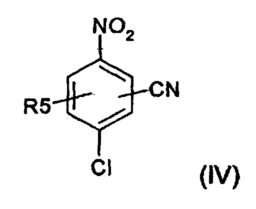

mit einem Amin der Formel HNR1R2, Reduktion der Nitrilgruppe, Alkylierung der Aminogruppe mit einer Verbindung der Formel (V) und anschliessende Reduktion der Nitrogruppe; 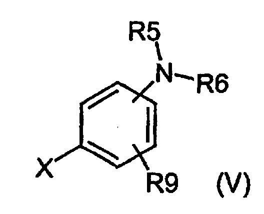 wobei in den Formeln (IV) und (V) die Restgruppen die folgende Bedeutung haben: **X** ist gleich einem Halogenatom und **R1,R2, R5, R6** und **R9** haben die gleiche Bedeutung wie in Formel (I).

Die erfindungsgemäßen 2,5-Diamino-1-aminomethylbenzol-Derivate der Formel (I) sind in Wasser löslich und ermöglichen Färbungen mit hoher Farbintensität und ausgezeichneter Farbechtheit, insbesondere was die Lichtechtheit, Waschechtheit und Reibeechtheit anbetrifft.

Ein weiterer Gegenstand der vorliegenden Erfindung ist daher weiterhin ein Mittel zum oxidativen Färben keratinischer Fasern, welches dadurch gekennzeichnet ist, dass es mindestens ein 2,5-Diamino-1-aminomethylbenzol-Derivat der Formel (I) enthält.

Das 2,5-Diamino-1-aminomethylbenzol-Derivate der Formel (I) ist in dem erfindungsgemäßen Färbemittel in einer Menge von etwa 0,005 bis 20 Gewichtsprozent enthalten, wobei eine Menge von etwa 0,01 bis 10,0 Gewichtsprozent und insbesondere 0,1 bis 8 Gewichtsprozent bevorzugt ist.

Die Verbindungen der Formel (I) färben keratinisches Material ohne den Zusatz weiterer Farbstoffe in intensiv blauen Farbtönen. Zur Erzielung weiterer Farbnuancen können zusätzlich übliche oxidative Farbstoffe, beispielsweise Entwicklersubstanzen oder Kupplersubstanzen, alleine oder im Gemisch miteinander, zugesetzt werden.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy -2 -methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol oder 2,3-Indolindion in Betracht.

Als Entwicklersubstanzen kommen vorzugsweise 1,4-Diaminobenzol; 1,4-Diamino-2-methylbenzol; 1,4-Diamino-2,6-dimethylbenzol; 2,5-Diamino-1,3-diethylbenzol-dihydrochlorid; 1,4-Diamino-2,5-dimethylbenzol; 1,4-Diamtno-2,3-dimethyl-benzol; 2-Chlor-1,4-diaminobenzol; 1,4-Diamino-2-(thiophen-2-yl)benzol-dihydrochlorid; 1,4-Diamino-2-(thiophen-3-yl)benzol-dihydrochlorid; 3-(2,5-Diaminophenyl)pyridintrihydrochlorid; 2,5-Diaminobiphenyl-dihydrochlorid; 1,4-Diamino-2-(methoxymethyl)benzol-dihydrochlorid; 1-(Aminomethyl)-2,5-diaminobenzol-dihydrochlorid; 1,4-Diamino-2-(hydroxymethyl)benzol; 1,4-Diamino-2-(2-hydroxyethoxy)benzol-dihydrochlorid; 2-(2-(Acetylamino)ethoxy)-1,4-diaminobenzol-dihydrochlorid; 4-Phenylamino-anilin; 4-Dimethylaminoanilin; 4-(Dipropylamino)anilin; 4-Diethylamino-anilin; 4-(Ethyl(2-hydroxyethyl)amino)anilin; 4-(Di(2-hydroxyethyl)amino)-anilin; 4-(Di(2-hydroxyethyl)amino)-2-methylanilin; 4-((2-Methoxyethyl)amino)-anilin; 4-((3-Hydroxypropyl)amino)-anilin; 4-((2,3-Dihydroxypropyl)amino)anilin; 1,4-Diamino-2-(2-hydroxyethyl)-benzol; 1,4-Diamino-2-(1-methylethyl)-benzol; 1,3-Bis-((4-aminophenyl)(2-hydroxyethyl)amino)-2-propanol; 1,4-Di((4-Aminophenyl)amino)butan; 1,8-Bis-(2,5-diaminophenoxy)-3,6-dioxaoctan; 4-Aminophenol; 4-Amino-3-methyl-phenol; 4-Amino-3-(hydroxymethyl)phenol; 4-Amino-3-fluorphenol; 4-Methylamino-phenol; 4-Amino-2-(aminomethyl)phenol; 4-Amino-2-(hydroxymethyl)phenol; 4-Amino-2-fluorphenol; 4-Amino-2-((2-hydroxyethyl)amino)methyl-phenol; 4-Amino-2-methylphenol; 4-Amino-2-(methoxymethyl)-phenol; 4-Amino-2-(2-hydroxyethyl)-phenol; 5-Amino-salicylsäure ; 2,5-Diamino-pyridin; 2,4,5,6-Tetraamino-pyrimidin; 2,5,6-Triamino-4(1H)-pyrimidon; 4,5-Diamino-1-(2-hydroxyethyl)-1H-pyrazol; 4,5-Diamino-1-(1-methylethyl)-1Hpyrazol; 4,5-Diamino-1-((4-methylphenyl)methyl)-1H-pyrazol; 1-((4-Chlorphenyl)methyl)-4,5-diamino-1H-pyrazol oder 4,5-Diamino-1-methyl-1 H-pyrazol in Betracht.

Weiterhin kann das erfindungsgemäße Färbemittel zusätzlich andere Farbkomponenten, beispielsweise 2-Aminophenol, 2-Amino-6-methylphenol und 2-Amino-5-methylphenol, sowie femer übliche direktziehende Farbstoffe, zum Beispiel Triphenylmethanfarbstoffe wie 4-[(4'-aminophenyl)-(4'imino-2",5"-cyclohexadien-1"-yliden)-methyl]-2-methylaminobenzol-monohydrochlorid (C.I. 42 510) und 4-[(4'amino-3'methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie-4-(2'-hydroxyethyl-)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)-azo]-5hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraamino-antrachinon, enthalten. Das Färbemittel kann diese Farbkomponenten in einer Menge von etwa 0,1 bis 4,0 Gewichtsprozent enthalten. So ist es beispielsweise durch Verwendung einer Kombination der Verbindungen der Formel (I) mit 2-Aminophenol, 2-Amino-6-methylphenol oder 2-Amino-5-methyl-phenol möglich, blond bis braune Haarfärbungen zu erzielen.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein. Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel. schwach sauer, neutral oder alkalisch reagieren. Insbesondere weist es einen pH-Wert von 6,0 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren -Bereichkommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3- bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man lässt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschliessend wird das Haar getrocknet.

Die erfindungsgemäßen Färbemittel mit einem Gehalt an 2,5-Diaminp-1-aminomethylbenzol -Derivaten der Formel (I) ermöglichen Färbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Färbemittel je nach Art und Zusammensetzung der Farbkomponenten eine breite Palette verschiedener Farbnuancen, welche sich von blonden über braune, purpure, violette bis hin zu blauen und schwarzen Farbtönen erstreckt. Hierbei zeichnen sich die Farbtöne durch ihre besondere Farbintensität aus. Die sehr guten färberischen Eigenschaften der erfindungsgemässen Färbemittel zeigen sich weiterhin darin, daß diese Mittel eine Anfärbung von ergrauten, chemisch nicht vorgeschädigten Haaren problemlos und mit guter Deckkraft ermöglichen. Die Verbindungen der Formel (I) ermöglichen zudem eine intensiv blaue Färbung von Keratinfasem ohne den Zusatz weiterer Farbstoffe.

Die nachfolgenden Beispiele sollen den Gegenstand der Erfindung näher erläutern, ohne ihn darauf zu beschränken.

### Beispiele

### Herstellungsbeispiele

### Beispiele 1: Synthese von 2.5-Diamino-1-aminomethylbenzol -Derivaten der Forme (I) (Allgemeine Synthesevorschrift)

### A. Synthese von 2.5-Bis-tert.-butyloxycarbonvlamino-brombenzol

15,65 g (0,07 mol) Brom-p-phenylenlendiamin-Hydrochlorid und 32,7 g (0,15 mol) Di-tert.-butyl-dicarbonat werden in einer Mischung von 250 ml 2N Natriumhydroxidlösung und 250 ml Trifluortoluol gelöst und auf 45°C erwärmt. Die Reaktionmischung wird 3 Tage gerührt. Schrittweise werden weitere 30 g (0,14 mol) Di-tert.-butyl-dicarbonat zugegeben.

Anschliessend wird die organische Schicht abgetrennt und die wässrige Phase zweimal mit 100ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-Bis-tert.-butyloxycarbonylaminobrombenzol mit einem Schmelzpunkt von 130 °C erhalten.

### B. Synthese von N-(4-tert.Butyloxycarbonylamino-2-formyl-phenyl)-carbaminsäure-tert.butylester

3,3 g (0,01 mol) 2,5-Bis-tert.-butyloxycarbonylamino-brombenzol aus Stufe **A** werden unter Argon in 100 ml wasserfreiem Tetrahydrofuran gelöst. Schrittweise werden 17 ml einer 1,6molaren etherischen Methyllithiumlösung (0,03 mol) zugegeben. Die Reaktionsmischung wird auf -20°C abgekühlt. Anschliessend werden schrittweise 7 ml einer 1,5 molaren t-Butyllithiumlösung (0,01 mol) zugegeben. Nach beendeter Zugabe wird die Lösung 30 Minuten lang bei -20°C gerührt. Anschliessend werden 1,2 g Dimethylformamid (0,02 mol) zugegeben und die Reaktionmischung eine Stunde lang bei -20°C gerührt. Nach langsamer Erwämung auf Raumtemperatur wird die Reaktionsmischung mit Wasser hydrolisiert und dann auf Ether gegossen, die wässerige Phase mit Ether extrahiert und sodann die organische Phase mit Magnesiumsulfat getrocknet. Das Lösungsmittel wird am Rotationsverdampfer abdestilliert und der Rückstand an Kieselgel mit Petrolether/Essigsäureethylester (9:1) gereinigt.

### C. Synthese von 2,5-Diamino-1-aminomethylbenzolen

0,033 g (0,0001 mol) N-(4-tert.Butyloxycarbonylamino-2-formyl-phenyl)-carbaminsäure-tert.butylester aus Stufe **B** und 0,00015 mol des entsprechenden Amins werden in 1,2-Dichlorethan gelöst. Anschliessend werden 0,1 ml einer Essigsäurelösung (1m, in 1,2-Dichlorethan) und 0,06 g NaBH(OAc)₃ (0,0003 mol) zugegeben und die Reaktionmischung wird 5 bis 15 Stunden bei Raumtemperatur gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) chromatographisch gereinigt. Das so erhaltene Produkt wird in 4 ml Ethanol auf 50 °C erwärmt. Anschliessend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.

### a. 4-(2,5-Diamino-benzylamino)-anilin Hydrochlorid

Verwendetes Amin: 4-tert.-Butyloxycarbonylamino-anilin
Ausbeute: 0,025 g (67 % der Theorie)
Masspektrum: MH⁺ 229 (100)

### b. 2-(4-Amino-2-methyl-phenyl)aminomethyl-1,4-diamino-benzol Hydrochlorid und 2-(4-Amino-3-methyl-phenyl)aminomethyl-1,4-diamino-benzol Hydrochlorid

Verwendetes Amin: 4-tert.-Butyloxycarbonylamino-3-methyl-anilin beziehungsweise 4-tert.-Butyloxycarbonylamino-2-methyl-anilin
Ausbeute: 0.021 g (27 % der Theorie)
Masspektrum: MH⁺ 243 (80)

### c. 2-(3-Amino-phenyl)aminomethyl-1,4-diamino-benzol Hydrochlorid

Verwendetes Amin: 3-tert.-Butyloxycarbonylamino-anilin
Ausbeute: 0,025 g ( 67 % der Theorie)
Masspektrum: MH⁺ 229 (100)

### d. 2-[5-Amino-2-(2,5-diamino-benzylamino)-phenyl]-ethanol Hydrochlorid und 2-[2-Amino-5-(2,5-diamino-benzylamino)-phenyl]-ethanol Hydrochlorid

Verwendetes Amin: 4-tert.-Butyloxycarbonylamino-3-(2-hydroxyethyl)-anilin beziehungsweise 4-tert.-Butyloxycarbonylamino-2-(2-hydroxyethyl)-anilin
Ausbeute: 0,025 g (30 % der Theorie)
Masspektrum: MH⁺ 273 (100)

### e. 2-[4-Amino-2-(2,5-diamino-benzylamino)-phenoxy]-ethanol Hydrochlorid

Verwendetes Amin: 4-tert.-Butyloxycarbonylamino-2-amino-(2-hydroxy)-ethoxy-benzol
Ausbeute: 0,025 g (58 % der Theorie)
Masspektrum: MH⁺ 289 (100)

### f. 2-(4-Dimethylamino-phenylaminomethyl-1,4-diamino-benzol Hydrochlorid

Verwendetes Amin: 4-Amino-N,N-dimethylanilin
Ausbeute: 0,025 g (62 % der Theorie)
Masspektrum: MH⁺ 257 (100)

**Tabelle 1:**

| (in den Beispielen 3-17 verwendete Abkürzung für die Farbstoffe) | |
|---|---|
| **2,5-Diamino-1-aminomethylbenzol -Derivate der Formel (I)** | |
| **E1** ^{**a**} | 4-(2,5-Diamino-benzylamino)-anilin Hydrochlorid |
| **E1** ^{**b**} | 2-(4-Amino-2-methyl-phenyl)aminomethyl-1,4-diaminobenzol Hydrochlorid / 2-(4-Amino-3-methyl-phenyl)-aminomethyl-1,4-diamino-benzol Hydrochlorid |
| **E1** ^{**c**} | 2-(3-Amino-phenyl)aminomethyl-1 ,4-diamino-benzol Hydrochlorid |
| **E1** ^{**d**} | 2-[5-Amino-2-(2,5-diamino-benzylamino)-phenyl]-ethanol Hydrochlorid / 2-[2-Amino-5-(2,5-diamino-benzylamino) -phenyl]-ethanol Hydrochlorid |
| **E1** ^{**e**} | 2-[4-Amino-2-(2,5-diamino-benzylamino)-phenoxy]-ethanol Hydrochlorid |
| **E1** ^{**f**} | 2-(4-Dimethylamino-phenylaminomethyl-1,4-diamino-benzol Hydrochlorid |
| | |

| **Entwicklersubstanzen** | |
|---|---|
| **E2** | 1,4-Diaminobenzol |
| **E3** | 2,5-Diamino-phenylethanol-sulfat |
| **E9** | 2,5-Diaminotoluol-sulfat |
| | |

| **Kupplersubstanzen** | |
|---|---|
| **K13** | 1,3-Diamino-4-(2'-Hydroxyethoxy)benzol-sulfat |
| **K22** | 5-Amino-2-methyl-phenol |
| **K25** | 1-Naphthol |
| **K31** | 1,3-Dihydroxy-benzol |
| | |

| **Farbstoffe** | |
|---|---|
| **D1** | 2-Amino-5-methylphenol |
| **D2** | 2-Amino-6-methylphenol |
| **D5** | 2-Amino-6-chlor-4-nitro-phenol |

### Beispiele 3 bis 11: Haarfärbemittel

| Haafärbelösungen | |
|---|---|
| X g | 4-(2,5-Diamino-benzylamino)-anilin Hydrochlorid (E1^{a}) |
| U g | Entwicklersubstanz E2 bis E9 gemäß der nachfolgenden Tabelle 2 |
| Z g | Farbstoff D gemäß der nachfolgenden Tabelle 2 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40°C wird das Haar mit Wasser gespült, mit einem handelsüblichen Shampoo gewaschen und getrocknet. Die Färbeergebnisse sind der nachfolgenden Tabellen 2 zu entnehmen.

**Tabelle 2:**

| **Beispiel Nr.** | **3** | **4** | **5** | **5** | **7** | **8** | **9** | **10** | **11** |
|---|---|---|---|---|---|---|---|---|---|
| **Farbstoffe** | **Einsatzmenge der Farbstoffe in g** | | | | | | | | |
| **E1**^{**a**} | 0,90 | 0,37 | 0,30 | 0,38 | 0,38 | 0,38 | 0,38 | 0,38 | 0,30 |
| **E2** | | | | 0,12 | | | | | |
| **E3** | | | | | 0,15 | | | | |
| **E9** | | | | | | 0,13 | | | |
| **D1** | | 0,18 | | 0,18 | 0,18 | 0,18 | 0,06 | 0,18 | 0,30 |
| **D2** | | | 0,18 | | | | 0,24 | | |
| **D5** | | | | | | | | 0,12 | |
| **Farbe** | **tiefblau** | **mittelbraun** | **mittelblond** | **schwarzbraun** | **braun** | **scwarzbraun** | **mittelbraun** | **braun** | **rotbraun** |

### Beispiele 12 bis 17: Haarfärbemittel

Es werden Haarfärbelösungen der folgenden Zusammensetzung hergestellt:

| | |
|---|---|
| 0,00125 mol | 2,5-Diamino-1-aminomethylbenzol -Derivate der Formel (I) gemäß der nachfolgenden Tabelle 3 (E1^{a}-E1^{f}) |
| 0,00125 mol | Kupplersubstanz K gemäß Tabelle 3 |
| 1,0 g | Kaliumoleat (8prozentige wäßrige Lösung) |
| 1,0 g | Ammoniak (22prozentige wäßrige Lösung) |
| 1,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen Wasserstoffperoxidlösung vermischt. Anschliessend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 3 zusammengefasst.

**Tabelle 3:**

| **Beispiel Nr.** | **Entwicklersubstanz** | **Kupplersubstanz / erhaltene Färbung** | | | |
|---|---|---|---|---|---|
| | | **K31** | **K13** | **K22** | **K25** |
| **12** | **E1**^{**a**} | blau | tiefblau | blau | tiefblau |
| **13** | **E1**^{**b**} | violett | tiefblau | violett | blau |
| **14** | **E1**^{**c**} | tiefblau | tiefblau | graurosa | graublau |
| **15** | **E1**^{**d**} | violett | tiefblau | violett | violett |
| **16** | **E1**^{**e**} | graublau | graublau | graublau | graublau |
| **17** | **E1**^{**f**} | hell-aschblond | graublau | purpur | graurosa |

### Beispiel 18: Haarfärbemittel

| | |
|---|---|
| 0,32 g | 2,5-Diamino-toluol Hydrochlorid |
| 0,04 g | 5-Amino-2-methylphenol |
| 0,09 g | 4-(2,5-Diamino-benzylamino)-anilin Hydrochlorid |
| 0,03 g | 3-Aminophenol |
| 0,03 g | 1,3-Dihydroxy-benzol |
| 0,04 g | 1,3-Dihydroxy-2-methylbenzol |
| 0,10 g | 4-Amino-3-methylphenol |
| 0,20 g | 2-Amino-5-methylphenol |
| 0,10 g | 2-Amino-6-methylphenol Hydrochlorid |
| 0,01 g | 4-Chlor-N-(2-hydroxyethyl)-2-nitroanilin |
| 0,02 g | 2-Amino-4,6-dinitrophenol |
| 0,10 g | 2-Chlor-6-(ethylamino)-4-nitrophenol |
| 10,00 g | Kaliumoleat (8prozentige wässrige Lösung) |
| 10,00 g | Ammoniak (22prozentige wässrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine braune Färbung erhalten.

### Beispiel 19: Haarfärbemittel

| | |
|---|---|
| 0,32 g | 2,5-Diamino-phenylethanol-sulfat |
| 0,04 g | 5-Amino-2-methylphenol |
| 0,05 g | 4-(2,5-Diamino-benzylamino)-anilin Hydrochlorid |
| 0,03 g | 3-Aminophenol |
| 0,03 g | 1 ,3-Dihydroxy-benzol |
| 0,04 g | 1,3-Dihydroxy-2-methylbenzol |
| 0,10 g | 4-Amino-3-methylphenol |
| 0,20 g | 2-Amino-5-methylphenol |
| 0,10 g | 2-Amino-6-methylphenol Hydrochlorid |
| 0,01 g | 4-Chlor-N-(2-hydroxyethyl)-2-nitroanilin |
| 0,02 g | 2-Amino-4,6-dinitrophenol |
| 0,10 g | 2-Chlor-6-(ethylamino)-4-nitrophenol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Das Haar hat eine braune Färbung erhalten.

Alle Prozentangaben stellen soweit nicht anders angegeben Gewichtsprozente dar.

## Patentansprüche

1. 2,5-Diamino-1-aminomethylbenzol-Derivate der allgemeinen Formel (I) oder deren physiologisch verträgliche, wasserlösliche Salze, 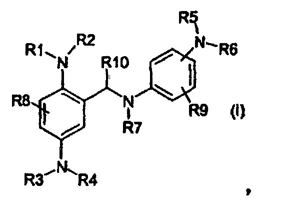 worin
**R1,R2,R3,R4,R5,R6 und R7** unabhängig voneinander Wasserstoff, eine C₁-C₆- Alkylgruppe, eine C₁-C₄-Hydroxyalkylgruppe, eine C₂-C₄-Dihydroxyalkylgruppe oder eine C₁-C₄-Alkoxy-(C₁-C₂)-alkylgruppe darstellen oder R1 und R2 und/oder R3 und R4 und/oder R5 und R6 gemeinsam einen viergliedrigen bis achtgliedrigen aliphatischen Ring bilden, unter der Bedingung, dass mindestens 2 der Reste R1 bis R6 Wasserstoff darstellen;
**R8** gleich Wasserstoff, einem Halogenatom, einer C₁-C₄-Alkylgruppe, einer C₁-C₄ -Hydroxyalkylgruppe oder einer C₁-C₄-Alkoxygruppe ist;
**R9** Wasserstoff, ein Halogenatom, eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄-Alkylthioethergruppe, eine Mercaptogruppe, eine Aminogruppe, eine C₁-C₄-Alkylaminogruppe, eine C₁-C₄-Hydroxyalkyl-aminogruppe, eine Di(C₁-C₄)-alkylaminogruppe, eine Di(C₁-C₄-hydroxyalkyl)-aminogruppe, eine (Dihydroxy(C₂-C₄)-alkyl)aminogruppe, eine (C₁-C₄-Hydroxyalkyl)-C₁-C₄-alkylaminogruppe, eine Trifluormethangruppe, eine C₁-C₄-Hydroxyalkylgruppe oder eine C₃-C₄-Dihydroxy-alkylgruppe bedeutet; und
**R10** gleich Wasserstoff oder einer C₁-C₆ Alkylgruppe Gruppe ist.

2. 2,5-Diamino-1-aminomethylbenzol-Derivat der Formel (I) gemäss Anspruch 1, **dadurch gekennzeichnet, dass** einer oder mehrere der Reste **R5** bis **R10** gleich Wasserstoff sind.

3. 2,5-Diamino-1-aminomethylbenzol-Derivat der Formel (I) gemäss Anspruch 1 oder 2, **dadurch gekennzeichnet, dass** die Reste **R1, R2, R3** und **R4** gleich Wasserstoff sind.

4. 2,5-Diamino-1-aminomethylbenzol-Derivat der Formel (I) gemäss einem der Ansprüche 1 bis 3, **dadurch gekennzeichnet, dass R7, R8, R9** und **R10** gleich Wasserstoff sind und **R5** und **R6** unabhänging voneinander gleich Wasserstoff, einer C₁-C₄-Alkylgruppe, einer C₁-C₄-Hydroxyalkylgruppe oder einer C₂-C₄-Dihydroxyalkylgruppe sind oder **R5** und R6 gemeinsam einen viergliedrigen Ring bilden.

5. 2,5-Diamino-1-aminomethylbenzol-Derivat der Formel (I) gemäss einem der Ansprüche 1 bis 4, **dadurch gekennzeichnet, dass** es ausgewählt ist aus der Gruppe bestehend aus 2-((2-Amino-phenylamino)-methyl)-1,4-diamino-benzol; 2-((3-Amino-phenylamino)-methyl)-1,4-diamino-benzol; 2-((4-Amino-phenylamino)-methyt)-1,4-diamino-benzol; 2-((4-Bis(2-hydroxyethyl)amino-phenyl-amino)-methyl)-1,4-diaminobenzol; 2-((4-Dimethylamino-phenylamino)-methyl)-1,4-diamino-benzol; N¹,N¹-Bis(hydroxyethyl)-2-((3-amino-phenylamino)-methyl)-1,4-diaminobenzol; N¹,N¹-Bis(hydroxyethyl)-2-((4-amino-phenylamino)-methyl)-1,4-diamino-benzol; N¹,N¹-Bis(hydroxyethyl)-2-((4-bis(2-hydroxyethyl)aminophenylamino)-methyl)-1,4-diamino-benzol; N¹-Dihydroxypropyl-2-((4-amino-phenylamino)-methyl)-1,4-diamino-benzol; N¹-Dihydroxypropyl-2-((4-bis(2-hydroxyethyl)amino-phenylamino)-methyl)-1,4-diamino-benzol; N¹-Dihydroxypropyl-2-((4-dimethylamino-phenylamino)-methyl)-1,4-diamino-benzol; N⁴,N⁴-Bis(hydroxyethyl)-2-((3-amino-phenylamino)-methyl)-1,4-diamino-benzol; N⁴,N⁴-Bis(hydroxyethyl)-2-((4-amino-phenylamino)-methyl)-1,4-diamino-benzol; N⁴,N⁴-Bis(hydroxy-ethyl)-2-((4-bis(2-hydroxyethyl)amino-phenylamino)-methyl)-1,4-diamino-benzol; N⁴,N⁴-Bis-(hydroxyethyl)-2-((4-dimethylamino-phenylamino)-methyl)-1,4-diaminobenzol; N⁴-Dihydroxypropyl-2-((4-amino-phenyl-amino)-methyl)-1,4-diamino-benzol; N⁴-Dihydroxypropyl-2-((4-bis(2-hydroxyethyl)amino-phenylamino)-methyl)-1,4-diamino-benzol; N⁴-Dihydroxypropyl-2-((4-dimethylamino-phenylamino)-methyl)-1,4-diamino-benzol; 2-((2-(2-Hydroxyethyl)-amino-phenylamino)-methyl)-1,4-diamino-benzol; 2-((2-(Pyrrolidin-1-yl)-phenylamino)-methyl)-1,4-diamino-benzol; 2-((2-Dihydroxypropylaminophenylamino)-methyl)-1,4-diamino-benzol; 2-((2-Methylamino-phenylamino)-methyl)-1,4-diamino-benzol; 2-((3-(2-Hydroxyethyl)amino-phenylamino)-methyl)-1,4-diamino-benzol; 2-((3-(Pyrrolidin-1-yl)-phenylamino)-methyl)-1,4-diamino-benzol; 2-((3-Dihydroxypropylamino-phenylamino)-methyl)-1,4-diamino-benzol; 2-((3-Methylamino-phenylamino)-methyl)-1,4-diamino-benzol; 2-((4-(2-Hydroxyethyl)amino-phenylamino)-methyl)-1,4-diamino-benzol; 2-((4-(Pyrrolidin-1-yl)-phenylamino)-methyl)-1,4-diaminobenzol; 2-((4-Dihydroxypropylamino-phenylamino)-methyl)-1,4-diaminobenzol; 2-((4-Methylamino-phenylamino)-methyl)-1,4-diamino-benzol; 2-((2-(2-Hydroxyethyl)-4-amino-phenylamino)-methyl)-1,4-diamino-benzol; 2-((2-(2-Hydroxyethyl)-4-bis(2-hydroxyethyl)amino-phenylamino)-methyl)-1,4-diamino-benzol; 2-((2-Chlor-4-amino-phenylamino)-methyl)-1,4-diamino-benzol; 2-((2-Chlor-4-bis(2-hydroxyethyl)amino-phenylamino)-methyl)-1,4-diamino-benzol; 2-((2-Methoxy-4-amino-phenylamino)-methyl)-1,4-diamino-benzol; 2-((2-Methoxy-4-bis(2-hydroxyethyl)aminophenylamino)-methyl)-1,4-diamino-benzol; 2-((2-Methyl-4-amino-phenylamino)-methyl)-1,4-diamino-benzol; 2-((2-Methyl-4-bis(2-hydroxy-ethyl)-amino-phenylamino)-methyl)-1,4-diamino-benzol; 2-((3-(2-Hydroxy-ethyl)-4-amino-phenylamino)-methyl)-1,4-diamino-benzol; 2-((3-(2-Hydroxyethyl)-4-bis(2-hydroxyethyl)amino-phenylamino)-methyl)-1,4-diaminobenzol; 2-((3-Chlor-4-amino-phenylamino)-methyl)-1,4-diamino-benzol; 2-((3-Chlor-4-bis(2-hydroxyethyl)amino-phenylamino)-methyl)-1,4-diaminobenzol; 2-((3-Methoxy-4-amino-phenylamino)-methyl)-1,4-diamino-benzol; 2-((3-Methoxy-4-bis(2-hydroxyethyl)amino-phenylamino)-methyl)-1,4-diamino-benzol; 2-((3-Methyl-4-amino-phenylamino)-methyl)-1,4-diaminobenzol; 2-((3-Methyl-4-bis(2-hydroxy-ethyl)amino-phenylamino)-methyl)-1,4-diamino-benzol; 2-((4-(2-Hydroxy-ethoxy)-3-amino-phenylamino)-methyl)-1,4-diamino-benzol; 2-((4-(2-Hydroxyethoxy)-3-bis(2-hydroxyethyl)amino-phenylamino)-methyl)-1,4-diamino-benzol; 2-((4-(2-Hydroxyethyl)-3-amino-phenylamino)-methyl)-1,4-diamino-benzol und 2-((4-(2-Hydroxyethyl)-3-bis(2-hydroxyethyl)amino-phenylamino)-methyl)-1,4-diamino-benzol.

6. Mittel zur Färbung keratinischer Fasern, **dadurch gekennzeichnet, dass** es mindestens ein 2,5-Diamino-1-aminomethylbenzol-Derivat der Formel (I) gemäss einem der Ansprüche 1 bis 5 enthält.

7. Mittel nach Anspruch 6, **dadurch gekennzeichnet, dass** es das 2,5-Diamino-1-aminomethylbenzol-Derivat der Formel (I) in einer Menge von 0,005 bis 20 Gewichtsprozent enthält.

8. Mittel nach Anspruch 6 oder 7, **dadurch gekennzeichnet, dass** es zusätzlich mindestens einen weiteren Farbstoff aus der Gruppe bestehend aus Entwicklersubstanzen, Kupplersubstanzen, 2-Aminophenol, 2-Amino-6-methylphenol, 2-Ammo-5-methylphenol und direktziehenden Farbstoffen enthält.

9. Mittel nach einem der Ansprüche 6 bis 8, **dadurch gekennzeichnet, dass** es ein Haarfärbemittel ist.

## Claims

1. 2,5-Diamino-1-aminomethylbenzene derivatives of the general formula (I) or physiologically compatible water-soluble salts thereof, 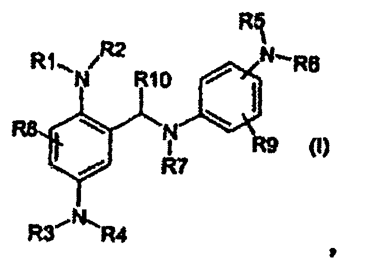 in which
**R1, R2, R3, R4, R5, R6 and R7,** independently of one another, are hydrogen, a C₁-C₆-alkyl group, a C₁-C₄-hydroxyalkyl group, a C₂-C₄-hydroxyalkyl group or a C₁-C₄-alkoxy-(C₁-C₂)-alkyl group, or R1 and R2 and/or R3 and R4 and/or R5 and R6 together form a four-membered to eight-membered aliphatic ring, with the proviso that at least 2 of the radicals R1 to R6 are hydrogen;
**R8** is hydrogen, a halogen atom, a C₁-C₄-alkyl group, a C₁-C₄-hydroxyalkyl group or a C₁-C₄-alkoxy group;
**R9** is hydrogen, a halogen atom, a cyano group, a hydroxyl group, a C₁-C₄-alkoxy group, a C₁-C₄-hydroxyalkoxy group, a C₁-C₆-alkyl group, a C₁-C₄-alkylthioether group, a mercapto group, an amino group, a C₁-C₄-alkylamino group, a C₁-C₄-hydroxyalkylamino group, a di (C₁-C₄)-alkylamino group, a di (C₁-C₄-hydroxyalkyl)amino group, a (dihydroxy(C₂-C₄)alkyl)amino group, a (C₁-C₄-hydroxyalkyl)-C₁-C₄-alkylamino group, a trifluoromethane group, a C₁-C₄-hydroxyalkyl group or a C₃-C₄-dihydroxyalkyl group; and
**R10** is hydrogen or a C₁-C₆-alkyl group.

2. 2,5-Diamino-1-aminomethylbenzene derivative of the formula (I) according to Claim 1, **characterized in that** one or more of the radicals **R5** to **R10** are hydrogen.

3. 2,5-Diamino-1-aminomethylbenzene derivative of the formula (I) according to Claim 1 or 2, **characterized in that** the radicals **R1, R2, R3** and **R4** are hydrogen.

4. 2,5-Diamino-1-aminomethylbenzene derivative of the formula (I) according to one of Claims 1 to 3, **characterized in that R7, R8, R9** and **R10** are hydrogen, and **R5** and **R6,** independently of one another, are hydrogen, a C₁-C₄-alkyl group, a C₁-C₄-hydroxyalkyl group or a C₂-C₄-dihydroxyalkyl group, or **R5** and **R6** together form a four-membered ring.

5. 2,5-Diamino-1-aminomethylbenzene derivative of the formula (I) according to one of Claims 1 to 4, **characterized in that** it is chosen from the group consisting of 2-((2-aminophenylamino)methyl)-1,4-diaminobenzene; 2-((3-aminophenylamino)methyl)-1,4-diaminobenzene; 2-((4-aminophenylamino)methyl)-1,4-diaminobenzene; 2-((4-bis(2-hydroxyethyl)aminophenylamino)methyl) -1,4-diaminobenzene; 2-((4-dimethylaminophenylamino )methyl)-1,4-diaminobenzene; N¹,N¹-bis-(hydroxyethyl)-2-((3-aminophenylamino)methyl)-1,4-diaminobenzene; N¹,N¹-bis(hydroxyethyl)-2-((4-aminophenylamino)methyl)-1,4-diaminobenzene; N¹,N¹-bis(hydroxyethyl)-2-((4-bis(2-hydroxyethyl)aminophenylamino)methyl)-1,4-diaminobenzene; N¹-dihydroxypropyl-2-((4-aminophenylamino)methyl)-1,4-diaminobenzene; N¹-dihydroxypropyl-2-((4-bis(2-hydroxyethyl)aminophenylamino)methyl)-1,4-diaminobenzene; N¹-dihydroxypropyl-2-((4-dimethylaminophenylamino)methyl)-1,4-diaminobenzene; N⁴,N⁴-bis(hydroxyethyl)-2-((3-aminophenylamino)methyl)-1,4-diaminobenzene; N⁴,N⁴-bis(hydroxyethyl)-2-((4-aminophenylamino)methyl)-1,4-diaminobenzene; N⁴,N⁴-bis(hydroxyethyl)-2-((4-bis(2-hydroxyethyl)aminophenylamino)methyl)-1,4-diaminobenzene; N⁴,N⁴-bis(hydroxyethyl)-2-((4-dimethylaminophenylamino)-methyl)-1,4-diaminobenzene; N⁴-dihydroxypropyl-2-((4-aminophenylamino)methyl)-1,4-diaminobenzene; N⁴-dihydroxypropyl-2-((4-bis(2-hydroxyethyl)aminophenylamino)methyl)-1,4-diaminobenzene; N⁴-dihydroxypropyl-2-((4-dimethylaminophenylamino)methyl)-1,4-diaminobenzene; 2-((2-(2-hydroxyethyl)aminophenylamino)-methyl)1,4-diaminobenzene; (2-((2-(pyrrolidin-1-yl)phenylamino)methyl)-1,4-diaminobenzene; 2-((2-dihydroxypropylaminophenylamino)methyl)-1,4-diaminobenzene; 2-((2-methylaminophenylamino)methyl)-1,4-diaminobenzene; 2-((3-hydroxyethyl)aminophenylamino)-methyl)-1,4-diaminobenzene; 2-((3-pyrrolidin-1-yl)phenylamino)methyl)-1,4-diaminobenzene; 2-((3-dihydroxypropylaminophenylamino)methyl)-1,4-diaminobenzene; 2-((3-methylaminophenylamino)methyl)-1,4-diaminobenzene; 2-((4-(2-hydroxyethyl)aminophenylamino)methyl)-1,4-diaminobenzene; 2-((4-(pyrrolidin-1-yl)phenylamino)methyl)-1,4-diaminobenzene; 2-((4-dihydroxypropylaminophenylamino)methyl)-1,4-diaminobenzene; 2-((4-methylaminophenylamino)methyl)-1,4-diaminobenzene; 2-((2-(2-hydroxyethyl)-4-aminophenylamino)methyl)-1,4-diaminobenzene; 2-((2-(2-hydroxyethyl)-4-bis(2-hydroxyethyl)aminophenylamino)-methyl)-1,4-diaminobenzene; 2-((-2-chloro -4-aminophenylamino)methyl)-1,4-diaminobenzene; 2-((2-chloro-4-bis(2-hydroxyethyl)aminophenylamino)methyl)-1,4-diamino benzene; 2-((2-methoxy-4-aminophenylamino)methyl)-1,4-diaminobenzene; 2-((2-methoxy-4-bis(2-hydroxyethyl)-aminophenylamino)methyl)-1,4-diaminobenzene; 2-((2-methyl-4-aminophenylamino)methyl)-1,4-diaminobenzene; 2-((2-methyl-4-bis(2-hydroxyethyl)aminophenylamino)-methyl)-1,4-diaminobenzene; 2-((3-(2-hydroxyethyl)-4-aminophenylamino)methyl)-1,4-diaminobenzene; 2-((3-(2-hydroxyethyl)-4-bis(2-hydroxyethyl)aminophenylamino)-methyl)-1,4-diaminobenzene; 2-((3-chloro-4-aminophenylamino)methyl)-1,4-diaminobenzene; 2-((3-chloro-4-bis(2-hydroxyethyl)aminophenylamino)methyl)-1,4-diaminobenzene; 2-((3-methoxy-4-aminophenylamino)methyl)-1,4-diaminobenzene; 2-((3-methoxy-4-bis(2-hydroxyethyl)-aminophenylamino)methyl)-1,4-diaminobenzene; 2-((3-methyl-4-aminophenylamino)methyl)-1,4-diaminobenzene; 2-((3-methyl-4-bis(2-hydroxyethyl)aminophenylamino)-methyl)-1,4-diaminobenzene; 2-((4-(2-hydroxyethoxy)-3-aminophenylamino)methyl)-1,4-diaminobenzene; 2-((4-(2-hydroxyethoxy)-3-bis(2-hydroxyethyl)aminophenylamino)-methyl)-1,4-diaminobenzene; 2-((4-(2-hydroxyethyl)-3-aminophenylamino)methyl)-1,4-diaminobenzene and 2-((4-(2-hydroxyethyl)-3-bis(2-hydroxyethyl)aminophenylamino)methyl)-1,4-diaminobenzene.

6. Agent for colouring keratin fibres, **characterized in that** it comprises at least one 2,5-diamino-1-aminomethylbenzene derivative of the formula (I) according to one of Claims 1 to 5.

7. Agent according to Claim 6, **characterized in that** it comprises the 2,5-diamino-l-aminomethylbenzene derivative of the formula (I) in an amount of from 0.005 to 20% by weight.

8. Agent according to Claim 6 or 7, **characterized in that** it additionally comprises at least one further dye from the group consisting of developer substances, coupler substances, 2-aminophenol, 2-amino-6-methylphenol, 2-amino-5-methylphenol and direct dyes.

9. Agent according to one of Claims 6 to 8, **characterized in that** it is a hair-colouring agent.

## Revendications

1. Dérivés de 2,5-diamino-1-aminométhylbenzène de formule générale (I) 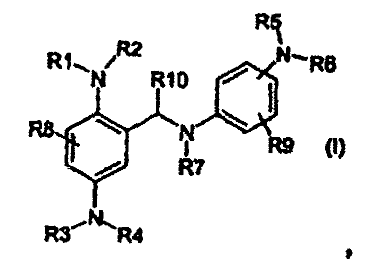 dans laquelle R1, R2, R3, R4, R5, R6 et R7 représentent, indépendamment les uns des autres, un atome d'hydrogène, un groupe alkyle en C₁-C₆, un groupe hydroxyalkyle en C₁-C₄, un groupe dihydroxyalkyle en C₂-C₄ ou un groupe alcoxy(C₁-C₄)-alkyle(C₁-C₂), ou R1 et R2 et/ou R3 et R4 et/ou R5 et R6 forment ensemble un cycle aliphatique à 4-8 chaînons, étant entendu qu'au moins deux des radicaux R1 à R6 représentent des atomes d'hydrogène ;
R8 est un atome d'hydrogène, un atome d'halogène, un groupe alkyle en C₁-C₄, un groupe hydroxyalkyle en C₁-C₄ ou un groupe alcoxy en C₁-C₄ ;
R9 représente un atome d'hydrogène, un atome d'halogène, un groupe cyano, un groupe hydroxy, un groupe alcoxy en C₁-C₄, un groupe hydroxyalcoxy en C₁-C₄, un groupe alkyle en C₁-C₆, un groupe alkyl(C₁-C₄)thioéther, un groupe mercapto, un groupe amino, un groupe alkyl(C₁-C₄)amino, un groupe hydroxyalkyl(C₁-C₄)amino, un groupe dialkyl(C₁-C₄)amino, un groupe di[hydroxyalkyl(C₁-C₄)]amino, un groupe di[hydroxyalkyl(C₂-C₄)]amino, un groupe [hydroxyalkyl(C₁-C₄)]-alkyl(C₁-C₄)amino, un groupe trifluorométhane, un groupe hydroxyalkyle en C₁-C₄ ou un groupe dihydroxyalkyle en C₃-C₄; et
R10 est un atome d'hydrogène ou un groupe alkyle en C₁-C₆,
ou sels solubles dans l'eau, physiologiquement acceptables de tels composés.

2. Dérivé de 2,5-diamino-1-aminométhylbenzène de formule (I) selon la revendication 1, **caractérisé en ce qu'**un ou plusieurs des radicaux R5 à R10 représentent un(des) atome(s) d'hydrogène.

3. Dérivé de 2,5-diamino-1-aminométhylbenzène de formule (I) selon la revendication 1 ou 2, **caractérisé en ce que** les radicaux R1, R2, R3 et R4 sont des atomes d'hydrogène.

4. Dérivé de 2,5-diamino-1-aminométhylbenzène de formule (I) selon l'une quelconque des revendications 1 à 3, **caractérisé en ce que** R7, R8, R9 et R10 sont des atomes d'hydrogène et R5 et R6 représentent, indépendamment l'un de l'autre, un atome d'hydrogène, un groupe alkyle en C₁-C₄, un groupe hydroxyalkyle en C₁-C₄ ou un groupe dihydroxyalkyle en C₂-C₄, ou R5 et R6 forment ensemble un cycle à 4 chaînons.

5. Dérivé de 2,5-diamino-1-aminométhylbenzène de formule (I) selon l'une quelconque des revendications 1 à 4, **caractérisé en ce qu'**il est choisi dans le groupe constitué par le 2-((2-aminophénylamino)méthyl)-1,4-diaminobenzène, le 2-((3-aminophénylamino)méthyl)-1,4-diaminobenzène, le 2-((4-aminophénylamino)méthyl)-1,4-diaminobenzène, le 2-((4-bis-(2-hydroxyéthyl)-aminophénylamino)méthyl)-1,4-diaminobenzène, le 2-((4-diméthylaminophénylamino)méthyl)-1,4-diaminobenzène, le N¹,N¹-bis(hydroxyéthyl)-2-((3-aminophénylamino)méthyl)-1,4-diaminobenzène, le N¹,N¹-bis(hydroxyéthyl)-2-((4-aminophénylamino)methyl)-1,4-diaminobenzène, le N¹,N¹-bis(hydroxyéthyl)-2-((4-bis(2-hydroxyéthyl)aminophénylamino)méthyl)-1,4-diaminobenzène, le N¹-dihydroxypropyl-2-((4-aminophénylamino)méthyl)-1,4-diaminobenzène, le N¹-dihydroxypropyl-2-((4-bis(2-hydroxyéthyl)aminophénylamino)méthyl)-1,4-diaminobenzène, le N¹-dihydroxypropyl-2-((4-diméthylaminophénylamino)méthyl)-1,4-diaminobenzène, le N⁴,N⁴-bis(hydroxyéthyl)-2-((3-aminophénylamino)méthyl)-1,4-diaminobenzène, le N⁴,N⁴-bis(hydroxyéthyl)-2-((4-aminophénylamino)méthyl)-1,4-diaminobenzène, le N⁴,N⁴-bis(hydroxyéthyl)-2-((4-bis(2-hydroxyéthyl)aminophénylamino)méthyl)-1,4-diaminobenzène, le N⁴,N⁴-bis(hydroxyéthyl)-2-((4-diméthylaminophénylamino)méthyl)-1,4-diaminobenzène, le N⁴-dihydroxypropyl-2-((4-aminophénylamino)méthyl)-1,4-diaminobenzène, le N⁴-dihydroxypropyl-2-((4-bis(2-hydroxyéthyl)aminophénylamino)méthyl)-1,4-diaminobenzène, le N⁴-dihydroxypropyl-2-((4-diméthylaminophénylamino)méthyl)-1,4-diaminobenzène, le 2-((2-(2-hydroxyéthyl)aminophénylamino)méthyl)-1,4-diaminobenzène, le 2-((2-(pyrrolidin-1-yl)phénylamino)-méthyl)-1,4-diaminobenzène, le 2-((2-dihydroxypropylaminophénylamino)méthyl)-1,4-diaminobenzène, le 2-((2-méthylaminophénylamino)méthyl)-1, 4-diaminobenzène, le 2-((3-(2-hydroxyéthyl)aminophénylamino)méthyl)-1,4-diaminobenzène, le 2-((3-(pyrrolidin-1-yl)phénylamino)méthyl)-1,4-diaminobenzène, le 2-((3-dihydroxypropylaminophénylamino)méthyl)-1,4-diaminobenzène, le 2-((3-méthylaminophénylamino)méthyl)-1,4-diaminobenzène, le 2-((4-(2-hydroxyéthyl)aminophénylamino)méthyl)-1,4-diaminobenzène, le 2-((4-(pyrrolidin-1-yl)phénylamino)méthyl)-1,4-diaminobenzène, le 2-((4-dihydroxypropylaminophénylamino)méthyl)-1,4-diaminobenzène, le 2-((4-méthylaminophénylamino)méthyl)-1,4-diaminobenzène, le 2-((2-(2-hydroxyéthyl)-4-aminophénylamino)méthyl)-1,4-diaminobenzène, le 2-((2-(2-hydroxyéthyl)-4-bis(2-hydroxyéthyl)aminophénylamino)méthyl)-1,4-diaminobenzène, le 2-((2-chloro-4-aminophénylamino)méthyl)-1,4-diaminobenzène, le 2-((2-chloro-4-bis(2-hydroxyéthyl)aminophénylamino)méthyl)-1,4-diaminobenzène, le 2-((2-méthoxy-4-aminophénylamino)méthyl)-1,4-diaminobenzène, le 2-((2-méthoxy-4-bis(2-hydroxyéthyl)aminophénylamino)méthyl)-1,4-diaminobenzène, le 2-((2-méthyl-4-aminophénylamino)méthyl)-1,4-diaminobenzène, le 2-((2-méthyl-4-bis(2-hydroxyéthyl)aminophénylamino)méthyl)-1,4-diaminobenzène, le 2-((3-(2-hydroxyéthyl)-4-aminophénylamino)méthyl)-1,4-diaminobenzène, le 2-((3-(2-hydroxyéthyl)-4-bis(2-hydroxyéthyl)aminophénylamino)méthyl)-1,4-diaminobenzène, le 2-((3-chloro-4-aminophénylamino)méthyl)-1,4-diaminobenzène, le 2-((3-chloro-4-bis(2-hydroxyéthyl)aminophénylamino)méthyl)-1,4-diaminobenzène, le 2-((3-méthoxy-4-aminophénylamino)méthyl)-1,4-diaminobenzène, le 2-((3-méthoxy-4-bis(2-hydroxyéthyl)aminophénylamino)méthyl)-1,4-diaminobenzène, le 2-((3-méthyl-4-aminophénylamino)méthyl)-1,4-diaminobenzène, le 2-((3-méthyl-4-bis(2-hydroxyéthyl)aminophénylamino)méthyl)-1,4-diaminobenzène, le 2-((4-(2-hydroxyéthoxy)-3-aminophénylamino)méthyl)-1,4-diaminobenzène, le 2-((4-(2-hydroxyéthoxy)-3-bis(2-hydroxyéthyl)aminophénylamino)méthyl)-1,4-diaminobenzène, le 2-((4-(2-hydroxyéthyl)-3-aminophénylamino)méthyl)-1,4-diaminobenzène et le 2-((4-(2-hydroxyéthyl)-3-bis(2-hydroxyéthyl)-aminophénylamino)méthyl)-1,4-diaminobenzène.

6. Produit pour la teinture de fibres contenant de la kératine, **caractérisé en ce qu'**il contient au moins un dérivé de 2,5-diamino-1-aminométhylbenzène de formule (I) selon l'une quelconque des revendications 1 à 5.

7. Produit selon la revendication 6, **caractérisé en ce qu'**il contient le dérivé de 2,5-diamino-1-aminométhylbenzène de formule (I) en une quantité de 0,005 à 20 % en poids.

8. Produit selon la revendication 6 ou 7, **caractérisé en ce qu'**il contient en outre au moins un autre colorant choisi dans le groupe constitué par des substances de type développeur, des substances de type copulant, le 2-aminophénol, le 2-amino-6-méthylphénol, le 2-amino-5-méthylphénol et des colorants directs.

9. Produit selon l'une quelconque des revendications 6 à 8, **caractérisé en ce qu'**il s'agit d'un produit de teinture pour cheveux.
